Europäisches Patentamt

European Patent Office

Office européen des brevets

(11) Publication number : **0 453 993 B1**

(12) **EUROPEAN PATENT SPECIFICATION**

(45) Date of publication of patent specification :
07.07.93 Bulletin 93/27

(51) Int. Cl.⁵ : **C07C 69/63, C07C 67/11**

(21) Application number : **91106328.7**

(22) Date of filing : **19.04.91**

(54) **Process for producing a halomethyl pivalate.**

(30) Priority : **20.04.90 JP 104544/90**

(43) Date of publication of application :
**30.10.91 Bulletin 91/44**

(45) Publication of the grant of the patent :
**07.07.93 Bulletin 93/27**

(84) Designated Contracting States :
**AT BE CH DE DK ES FR GB IT LI NL SE**

(56) References cited :
DE-A- 2 245 457
CHEMICAL ABSTRACTS, vol. 107, no. 17, 26
October 1987, Columbus, Ohio, US; S. A.
ANDREEV et al, "Chloromethyl pivalate",
page 664, abstract no. 153938q; & SU-A-1 313
850

(56) References cited :
CHEMICAL ABSTRACTS, vol. 101, no. 23, 3
December 1984, Columbus, Ohio, US; E. BIN-
DERUP et al, "Chlorosulfates as reagents in
the synthesis of carboxylic acid esters under
phase-transfer conditions", page 563, ab-
stract no. 210048b

(73) Proprietor : **IHARA CHEMICAL INDUSTRY Co.,
Ltd.**
**4-26, Ikenohata 1-chome**
**Taitoh-ku, Tokyo 110 (JP)**

(72) Inventor : **Yazawa, Naoto, Ihara Chemical
Industry Co., Ltd.**
**Kenkyusho, 2256, Nakanogo, Fujikawa-cho**
**Ihara-gun, Shizuoka-ken (JP)**
Inventor : **Ishikame, Keinosuke, Ihara
Chemical Ind. Co., Ltd.**
**4-26, Ikenohata 1-chome, Taitoh-ku**
**Tokyo (JP)**

(74) Representative : **Wächtershäuser, Günter, Dr.**
**Tal 29**
**W-8000 München 2 (DE)**

Jouve, 18, rue Saint-Denis, 75001 PARIS

## Description

The present invention relates to a process for producing a halomethyl pivalate which is useful as a starting material for cephem antibiotics (which are disclosed, for example, in Japanese Unexamined Patent Publication No. 19593/1987).

Heretofore, as a process for producing a halomethyl pivalate, it has been known to conduct so-called Blanc-Quelet reaction wherein formaldehyde and a hydrogen halide are reacted with pivalic acid in the presence of a catalyst such as aluminum chloride or zinc chloride. However, such a process has a problem that highly poisonous chloromethyl methyl ether and bis(chloromethyl)ether (hereinafter referred to simply as halomethyl ethers) are produced as by-products.

It is an object of the present invention to provide a process for producing a halomethyl pivalate which does not produce highly poisonous halomethyl ethers as by-products produced in conventional methods and which is useful for practical industrial application.

The present inventors have conducted extensive researches to develop a process for producing a halomethyl pivalate which does not produce highly poisonous halomethyl ethers. As a result, it has been found that the object of the present invention can be attained by using a dihalomethane having halogen atoms different from each other in a process for producing a halomethyl pivalate by reacting an aqueous solution of a metal salt of pivalic acid with a dihalomethane in the presence of a phase transfer catalyst. The present invention is based on this discovery.

Namely, the present invention provides a process for producing a halomethyl pivalate which comprises reacting an aqueous solution of a metal salt of pivalic acid with a dihalomethane selected from the group consisting of bromochloromethane, chloroiodomethane and bromoiodomethane in the presence of a phase transfer catalyst.

Now, the present invention will be described in detail with reference to the preferred embodiments.

As the aqueous solution of a metal salt of pivalic acid to be used as a starting material in the present invention, an aqueous solution of an alkali metal or alkaline earth metal salt of pivalic acid may be mentioned. As the alkali metal or alkaline earth metal salt of pivalic acid, sodium, potassium, magnesium or barium salt of pivalic acid may be mentioned.

Such a metal salt of pivalic acid can be readily produced by reacting pivalic acid with an aqueous solution of a hydroxide, carbonate or hydrogen carbonate of an alkali metal or alkaline earth metal. The aqueous solution of a metal salt of pivalic acid thus obtained may be used as it is in the form of the aqueous solution. Alternatively, pivalic acid and a metal salt of an alkali metal or alkaline earth metal are reacted directly in the reaction system to obtain an aqueous solution of the metal salt of pivalic acid.

Further, water is used at least in an amount capable of stirring, preferably at least in an amount capable of dissolving the inorganic salt formed.

Moreover, there is no particular restriction as to the order to add pivalic acid, the base, the dihalomethane and the phase transfer catalyst.

According to the present invention, it is essential to have a phase transfer catalyst present during the reaction of the aqueous solution of metal salt of pivalic acid with the dihalomethane. As the phase transfer catalyst, any catalyst which is usually called a phase transfer catalyst can be employed. For example, quaternary onium salts such as a quaternary ammonium salt, a quaternary pyridinium salt and a quaternary phosphonium salt are usually used. They may be used alone or in combination. Among them, preferred are tetrabutylammonium bromide, tetrabutylammonium chloride, trioctylmethylammonium chloride and trioctylmethylammonium bromide which are readily obtainable from the industrial viewpoint.

In the process of the present invention, the phase transfer catalyst is used in an amount of from 0.001 to 1 mol, preferably from 0.005 to 0.2 mol, per mol of the metal salt of pivalic acid.

Further, the dihalomethane selected from the group consisting of bromochloromethane, chloroiodomethane and bromoiodomethane, which is to be reacted with the aqueous solution of the metal salt of pivalic acid, is used in an amount of from 1 to 100 mols, preferably from 10 to 50 mols, per mol of the aqueous solution of the metal salt of pivalic acid.

In the process of the present invention, a non-aqueous inert organic solvent such as benzene, toluene and diethyl ether can be used as the case requires.

Now, a specific embodiment suitable for the process of the present invention, will be described. Firstly, a phase transfer catalyst, an aqueous solution of a metal salt of pivalic acid and a dihalomethane selected from the group consisting of bromochloromethane, chloroiodomethane and bromoiodomethane are mixed and reacted in predetermined proportions. There is no particular restriction on the reaction temperature, and it is usually from 0°C to the reflux temperature of the reaction system. The reaction time depends on the starting materials employed, the reaction temperature, the catalyst, etc., and it is usually about from 1 to 10 hours. The reaction can be conducted under atmospheric pressure or elevated pressure. After completion of the reaction, the reaction mixture is allowed to stand to have an aqueous phase and an oil phase separated. Then, only the oil phase is collected, washed and distilled off, whereby a halomethyl pivalate is obtained.

In the process of the present invention, the pivalic acid can be quantitatively recovered from the distillation residue by subjecting bis(pivaloyloxy)methane produced as by-product to hydrolysis with an alkali or acid, and the pivalic acid recovered can be reused. Accordingly, the process of the present invention is so efficient that the pivalic acid is consumed only for the formation of the halomethyl pivalate.

According to the process of the present invention wherein an aqueous solution of a metal salt of pivalic acid is reacted with a dihalomethane selected from the group consisting of bromochloromethane, chloroiodomethane and bromoiodomethane in the presence of a phase transfer catalyst, it is possible to produce halomethyl pivalate in good yield and without production of halomethyl ethers as by-products. Further, the pivalic acid can be recovered quantitatively by means of hydrolysis of bis(pivaloyloxy)methane produced as by-product, and the recovered pivalic acid can be reused. Thus, the process of the present invention is suitable as an industrial process for the production of halomethyl pivalate.

Now, the present invention will be described in further detail with reference to Examples. However, it should be understood that the present invention is by no means restricted by these specific Examples.

EXAMPLE 1

Into a 200 m$\ell$ reaction flask equipped with a condenser, a thermometer and a stirrer, 161.7 g of bromochloromethane, 5.1 g of pivalic acid, a 48% sodium hydroxide aqueous solution and 0.5 of tetrabutylammonium bromide were introduced and reacted for 2.5 hours. The conversion and the selectivity for chloromethyl pivalate are shown in Table 1. (Neither chloromethyl methyl ether nor bis(chloromethyl)ether was detected from the above reaction solution.)

After completion of the reaction, the reaction solution was allowed to stand, and the oil phase separated, was collected, washed and distilled off to obtain 153.6 g of bromochloromethane as a fraction having a boiling point of 67°C (yield: 95%). The distillation was further conducted under reduced pressure at a level of 66.5 mbar (50 mmHg) to obtain 2.58 g of chloromethyl pivalate as a fraction having a boiling point of from 70 to 72°C. The yield was 34.3% based on pivalic acid.

EXAMPLES 2-5

The reaction was conducted in the same manner as in Example 1 except that the amount of water, a 48% sodium hydroxide aqueous solution and tributylammonium bromide (TBAB) and the reaction temperature were changed as indicated in Table 1. The conversion of the reaction solution and the selectivity for chloromethyl pivalate are shown in Table 1.

## Table 1

| Example | 1 | 2 | 3 | 4 | 5 |
|---|---|---|---|---|---|
| $H_2O$ (g) | 10 | 10 | 25 | 10 | 10 |
| 48% NaOH (g) | 4.2 | 4.2 | 4.2 | 4.2 | 4.6 |
| TBAB[1] (g) | 0.5 | 0.5 | 0.5 | 0.3 | 0.5 |
| Reaction Temperature (°C) | 59-61 | 40 | 59-61 | 59-61 | 59-61 |
| Conversion (%) | 47.9 | 19.3 | 52.1 | 41.8 | 50.7 |
| Selectivity (%) | 71.3 | 88.4 | 71.1 | 75.3 | 69.8 |
| Selectivity for Bis-product[2] (%) | 28.4 | 11.6 | 28.9 | 24.7 | 30.2 |

1) Tributylammonium bromide
2) Bis(pivaloyloxy)methane

EXAMPLES 6-8

The reaction was conducted in the same manner as in Example 1 except that bases and catalysts as identified in Table 2 were used or a solvent was added. The conversion of the reaction solution and the selectivity for chloromethyl pivalate are shown in Table 2.

Table 2

| Example | 6 | 7 | 8 | COMPARA-TIVE EXAMPLE |
|---|---|---|---|---|
| $H_2O$ (g) | 10 | 10 | 10 | 10 |
| Base (g) | NaOH[1] 4.2 | $Na_2CO_3$ 2.7 | NaOH[1] 4.2 | NaOH[1] 4.2 |
| Catalyst (g) | TBAB[2] 0.5 | TBAB[2] 0.5 | TOMAC[3] 0.4 | TBAB[2] 0..5 |
| Solvent (g) | Toluene 10 g | | | |
| Reaction Temperature (°C) | 59-61 | 59-61 | 59-61 | 59-61 |
| Conversion (%) | 45.3 | 48.0 | 53.2 | 48.2 |
| Selectivity (%) | 72.0 | 70.4 | 68.7 | 0 |
| Selectivity for Bis-product[4] (%) | 28.0 | 29.6 | 31.2 | 100 |

1) 48% sodium hydroxide aqueous solution
2) Tributylammonium bromide
3) Trioctylmethylammonium chloride
4) Bis(pivaloyloxy)methane

In Tables 1 and 2, the selectivity and the selectivity for bis product were calculated as follows:

$$\text{Selectivity} = \frac{\text{Yield of halomethyl pivalate}}{\text{Conversion of pivalic acid}}$$

$$\text{Selectivity for bis - product} = \frac{\text{Yield of bis - product}}{\text{Conversion of pivalic acid}}$$

COMPARATIVE EXAMPLE

The reaction was conducted in the same manner as in Example 1 except that 217.3 g of dibromomethane was used instead of bromochloromethane. The results thus obtained are shown also in Table 2.

REFERENCE (Recovery of pivalic acid in Example 1)

The aqueous phase of the reaction solution was acidified with hydrochloric acid, and then extracted with diethyl ether. The extract was washed, dried and concentrated to obtain 2.6 g of the starting material pivalic acid (which corresponds to 51% of the pivalic acid introduced). Further, into the distillation residue, 5 g of a 24% sodium hydroxide aqueous solution was added and heated at 90°C for 2 hours under stirring. Then, the solution was cooled and acidified with hydrochloric acid. The solution was extracted with ethyl ether, and the extract was washed, dried and concentrated to obtain 0.7 g of the starting material pivalic acid (which corresponds to 13.7% based on the amount introduced).

Accordingly, 98.5% of pivalic acid which was not used for production of chloromethyl pivalate, was recovered.

## Claims

1. A process for producing a halomethyl pivalate which comprises reacting an aqueous solution of a metal salt of pivalic acid with a dihalomethane selected from the group consisting of bromochloromethane, chloroiodomethane and bromoiodomethane in the presence of a phase transfer catalyst.

2. The process according to Claim 1, wherein the phase transfer catalyst is at least one member selected from the group consisting of a quaternary ammonium salt, a quaternary pyridinium salt and a quaternary phosphonium salt.

3. The process according to Claim 1, wherein the phase transfer catalyst is at least one member selected from the group consisting of tetrabutylammonium bromide, tetrabutylammonium chloride, trioctylmethylammonium chloride and trioctylmethylammonium bromide.

4. The process according to Claim 1, wherein the metal salt is an alkali metal or alkaline earth metal salt.

5. The process according to Claim 1, wherein the phase transfer catalyst is used in an amount of from 0.001 to 1 mol per mol of the metal salt of pivalic acid.

6. The process according to Claim 1, wherein the dihalomethane is used in an amount of from 1 to 100 mols per mol of the metal salt of pivalic acid.

7. The process according to Claim 1, wherein the reaction is conducted at a temperature of from 0°C to the reflux temperature of the reaction system.

## Patentansprüche

1. Ein Verfahren zum Herstellen eines Halogenmethylpivalats, umfassend die Umsetzung einer wässerigen Lösung eines Metallsalzes von Pivalinsäure mit einem Dihalogenmethan, ausgewählt aus der Gruppe bestehend aus Bromchlormethan, Chlorjodmethan und Bromjodmethan, in Gegenwart eines Phasentransfer-Katalysators.

2. Das Verfahren nach Anspruch 1, worin der Phasentransfer-Katalysator mindestens einer ausgewählt aus der Gruppe bestehend aus einem quartären Ammoniumsalz, einem quartären Pyridiniumsalz und einem Phosphoniumsalz ist.

3. Das Verfahren nach Anspruch 1, worin der Phasentransfer-Katalysator mindestens einer ausgewählt aus der Gruppe bestehend aus Tetrabutylammoniumbromid, Tetrabutylammoniumchlorid, Trioctylmethylammoniumchlorid und Trioctylmethylammoniumbromid ist.

4. Das Verfahren nach Anspruch 1, worin das Metallsalz ein Alkali- oder Erdalkalimetallsalz ist.

5. Das Verfahren nach Anspruch 1, worin der Phasentransfer-Katalysator in einer Menge von 0,001 bis 1 Mol pro Mol des Metallsalzes der Pivalinsäure benutzt wird.

6. Das Verfahren nach Anspruch 1, worin das Dihalogenmethan in einer Menge von 1 bis 100 Mole pro Mol des Metallsalzes der Pivalinsäure benutzt wird.

7. Das Verfahren nach Anspruch 1, worin die Umsetzung bei einer Temperatur von 0°C bis zur Rückflußtemperatur des Reaktionssystems ausgeführt wird.

## Revendications

1. Procédé de fabrication d'un pivalate d'halométhyle, qui comprend la réaction d'une solution aqueuse d'un sel métallique de l'acide pivalique avec un dihalométhane choisi dans le groupe constitué par le bromochlorométhane, le chloroiodométhane et le bromoiodométhane en présence d'un catalyseur de transfert de phase.

2. Procédé selon la revendication 1, dans lequel le catalyseur de transfert de phase est au moins un élément choisi dans le groupe constitué par un sel d'ammonium quaternaire, un sel de pyridinium quaternaire et un sel de phosphonium quaternaire.

3. Procédé selon la revendication 1, dans lequel le catalyseur de transfert de phase est au moins un élément choisi dans le groupe constitué par le bromure de tétrabutyl ammonium, le chlorure de tétrabutyl ammonium, le chlorure de trioctyl méthyl ammonium et le bromure de trioctyl méthyl ammonium.

4. Procédé selon la revendication 1, dans lequel le sel métallique est un sel de métal alcalin ou de métal alcalino-terreux.

5. Procédé selon la revendication 1, dans lequel le catalyseur de transfert de phase est utilisé dans une quantité de 0,001 à 1 mole par mole du sel métallique de l'acide pivalique.

6. Procédé selon la revendication 1, dans lequel le dihalométhane est utilisé dans une quantité de 1 à 100 moles par mole du sel métallique de l'acide pivalique.

7. Procédé selon la revendication 1, dans lequel la réaction est conduite à une température de 0°C à la température de reflux du système réactionnel.